# EUROPEAN PATENT APPLICATION

(11) **EP 4 813 326 A1**
(43) Date of publication of application: **30.09.2026**
(21) Application number: 25166662.4
(22) Date of filing: 27.03.2025
(51) Int. Cl.: A61B 17/70

(54) **COUPLING DEVICE FOR COUPLING A ROD TO A BONE ANCHORING ELEMENT**

(71) Applicant: Biedermann Technologies GmbH & Co. KG, 78166 Donaueschingen (DE)
(72) Inventor: BIEDERMANN, Lutz, 78048 VS-Villingen (DE); FISCHER, Bernd, 79877 Friedenweiler (DE)
(74) Representative: Prüfer & Partner mbB Patentanwälte · Rechtsanwälte

(57) **Abstract**

A coupling device for coupling a rod to bone or to a vertebra is provided, the coupling device including a receiving part configured to be pivotably connectable to a head of a bone anchoring element, the receiving part having a first end and a second end, a central axis extending through the first end and the second end, a channel for receiving a rod and an accommodation space for accommodating the head, the accommodation space having a lower opening at the second end, an inner surface portion of the receiving part in the accommodation space providing a first seat portion for the head; and a retainer element configured to be arranged in the accommodation space and having an inner surface providing a second seat portion for the head; wherein the first seat portion of the receiving part and the second seat portion of the retainer element form a seat that permits the head to pivot relative to the receiving part.

## Description

The invention relates to a coupling device for coupling a rod to a bone anchoring element. In particular, the invention relates to a coupling device for providing a modular bone anchoring device.

Bone anchoring devices comprising a bone anchoring element for anchoring in bone or in a vertebra and a coupling device for coupling the anchoring element to a rod are used in orthopedic surgery, in particular in spinal surgery.

US 9,962, 207 B2 describes a polyaxial bone anchoring device that includes a receiving part configured to be pivotably connected to a head of an anchoring element, the receiving part having a channel for receiving a rod and an accommodation space having an opening for accommodating the head, and a sleeve-like insert piece configured to be positioned around a portion of the head in the receiving part and to pivot in the receiving part. The sleeve-like insert piece includes a first part and a separable second part configured to be connected with the first part by a connection structure configured to permit a translational movement between the first part and the second part in a direction transverse to the sleeve axis, while preventing movement between the first part and the second part in a direction parallel to the sleeve axis.

US 2006/0149240 A1 describes a polyaxial bone screw assembly including a threaded shank body having an upper capture structure, a head and a multi-piece retainer, articulating structure. The geometry of the retainer structure pieces corresponds and cooperates with the external geometry of the capture structure to frictionally envelope the retainer structure between the capture structure and an internal surface defining a cavity of the head. The retainer structure includes a substantially spherical surface that mates with the internal surface of the head providing a ball joint, enabling the head to be disposed at an angle relative to the shank body.

It is the object of the invention to provide an improved coupling device with a simplified structure. It is a further object to provide a bone anchoring device comprising such a coupling device and a bone anchoring element.

The object is solved by a coupling device of claim 1 and by a bone anchoring device of claim 16. Further developments are given in the dependent claims.

According to an aspect, a coupling device for coupling a rod to bone or a vertebra includes a receiving part configured to be pivotably connectable to a head of a bone anchoring element, the receiving part having a first end and a second end, a central axis extending through the first end and the second end, a channel for receiving the rod and an accommodation space for accommodating the head, the accommodation space having a lower opening at the second end. An inner surface portion of the receiving part in the accommodation space provides a first seat portion for the head. The coupling device further includes a retainer element configured to be arranged in the accommodation space and having an inner surface providing a second seat portion for the head. The first seat portion of the receiving part and the second seat portion of the retainer element form a seat that permits the head to pivot relative to the receiving part.

The coupling device comprises a single part retainer element. Hence, the coupling device has a reduced number of parts. This may be advantageous in terms of manufacturing the coupling device and/or in view of the interaction of the element of the coupling device and the bone anchoring element with each other. Since the first seat portion is provided by the inner surface portion of the receiving part directly, the stability of the bone anchoring device including such a coupling device may be increased compared to coupling devices with two or more retainer elements.

According to a further aspect, the coupling device may comprise a pressure element for exerting pressure on an inserted head to clamp and/or lock an angular position of the bone anchoring element relative to the receiving part. The pressure element may also provide a frictional clamping of the head of the bone anchoring element and may at the same time provide a pre-locking function that prevents removal of the head from the receiving part through the lower opening once the pressure element assumes a pre-locking position.

The coupling device or parts thereof may be manufactured with an additive manufacturing method. This allows to generate shapes of the parts that would be difficult to generate conventionally with a subtractive method such as lathing, cutting etc..

The head of the bone anchoring element is insertable into the receiving part from a bottom end of the receiving part. Thus, a modular polyaxial bone anchoring device may be provided, that permits to select a suitable bone anchoring element and to combine it with the receiving part prior to or during surgery. In particular, the bone anchoring element may be inserted into bone or a vertebra first and the coupling device can be mounted on the head of the bone anchoring element *in-situ.*

According to a further aspect, a coupling device for coupling a rod to bone includes a receiving part configured to be pivotably connectable to a head of a bone anchoring element, the receiving part having a first end and a second end, a central axis extending through the first end and the second end, a channel for receiving a rod and an accommodation space for accommodating the head, the accommodation space having a lower an opening at the second end; and a pressure element configured to exert pressure on an inserted head, wherein the pressure element comprises a recess for receiving a portion of the head; the coupling device further includes a retainer element configured to be arranged in the accommodation space and to assume a position in which the retainer element narrows the lower opening, wherein a wall portion of the pressure element that is configured to engage an inserted head is further configured to interact with the receiving part to clamp the head by friction and to hold the pressure element in a pre-locking position such that an inserted head is prevented from removal through the lower opening.

Thus, a wall portion of the pressure element provides for both functions, a frictional clamping of the head and a pre-locking function. Thereby, the pressure element may be designed shorter in the axial direction.

Further features and advantages will become apparent from the description of embodiments by means of the accompanying drawings.

In the drawings:
- Fig. 1:: shows a perspective exploded view of a bone anchoring device according to a first embodiment.
- Fig. 2:: shows a perspective view of the bone anchoring device of Fig. 1 in an assembled state.
- Fig. 3:: shows a cross-sectional view of the bone anchoring device of Figs. 1 and 2, the cross-section taken in a plane extending through the central axis and perpendicular to the rod axis of an inserted rod.
- Fig. 4:: shows a perspective view from a top of a receiving part of the bone anchoring device of Figs. 1 to 3.
- Fig. 5:: shows a perspective view from a bottom of the receiving part of Fig. 4.
- Fig. 6:: shows a top view of the receiving part of Fig. 5 with a sectional line A-A.
- Fig. 7:: shows a cross-sectional view of the receiving part of Figs. 4 to 6, the cross-section taken along line A-A in Fig. 6.
- Fig. 8:: shows a cross-sectional view of the receiving part along a plane extending through the central axis and perpendicular to the line A-A in Fig. 6.
- Fig. 9:: shows a perspective view from a top of a retainer element of the coupling device of Figs. 1 to 3.
- Fig. 10:: shows a perspective view from a bottom of the retainer element of Fig. 9.
- Fig. 11:: shows a top view of the retainer element of Figs. 9 and 10 with a sectional line B-B.
- Fig. 12:: shows a cross-sectional view of the retainer element of Figs. 9 to 11, the cross-section taken along the line B-B in Fig. 11.
- Fig. 13:: shows a side view of the retainer element of Figs. 9 to 12.
- Fig. 14:: shows a perspective view from a top of a pressure element of the coupling device of Figs. 1 to 3.
- Fig. 15:: shows a perspective view from a bottom of the pressure element of Fig. 14.
- Fig. 16:: shows a top view of the pressure element of Figs. 14 and 15 with a sectional line D-D.
- Fig. 17:: shows a cross-sectional view of the pressure element of Figs. 14 to 16, the cross-section taken along the line D-D in Fig. 16.
- Fig. 18:: shows a side view of the pressure element of Figs. 14 to 17.
- Figs. 19 to 21:: show perspective views of steps of mounting the retainer element to the receiving part of the coupling device of Figs. 1 to 3.
- Figs. 22 to 24:: show cross-sectional views of the steps shown in Figs. 19 to 21, respectively, of mounting the retainer element to the receiving part of the coupling device of Figs. 1 to 3, the cross-section taken in a plane extending through the central axis and perpendicular to the rod channel.
- Figs. 25 to 28:: show cross-sectional views o f steps of mounting the coupling device to a bone anchoring element.
- Fig. 29:: shows a cross-sectional view of the coupling device with inserted head of the bone anchoring element in a pre-locking configuration, the cross-section taken in a plane extending through the central axis and perpendicular to the axis of the rod channel.
- Fig. 30:: shows a cross-sectional view of the coupling device of Fig. 29 with inserted rod and fixation element in a locked configuration of the head in the receiving part.
- Fig. 31:: shows a cross-sectional view of a second embodiment of the coupling device and a second embodiment of a bone anchoring device, the cross-section taken in a plane extending trough the central axis and perpendicular to the rod axis of an inserted rod.
- Fig. 32:: shows a perspective view from a top of the receiving part of the coupling device of Fig. 31.
- Fig. 33:: shows a perspective view from a bottom of the receiving part of Fig. 32.
- Fig. 34:: shows a top view of the receiving part of Figs. 32 and 33 with a sectional line F-F.
- Fig. 35:: shows a cross-sectional view of a receiving part of Figs. 32 to 34, the cross-section taken along the line F-F of Fig. 34.
- Fig. 36:: shows a cross-sectional view of the receiving part of Figs. 32 to 35, the cross-section taken in a plane extending through the central axis and perpendicular to the line F-F in Fig. 34.
- Fig. 37:: shows a perspective view from a top of the pressure element of the coupling device of Fig. 31.
- Fig. 38:: shows a perspective view from a bottom of the pressure element of Fig. 37.
- Fig. 39:: shows a top view of the pressure element of Figs. 37 and 38 with a sectional line G-G.
- Fig. 40:: shows a cross-sectional view of the pressure element of Figs. 37 to 39, the cross-section taken in a plane along the line G-G in Fig. 39.
- Fig. 41:: shows a side view of the pressure element of Figs. 37 to 40.
- Figs. 42 to 45:: show cross-sectional views of steps of mounting the coupling device according to Fig. 31 with inserted pressure element and inserted retainer element in the head of a bone anchoring element.

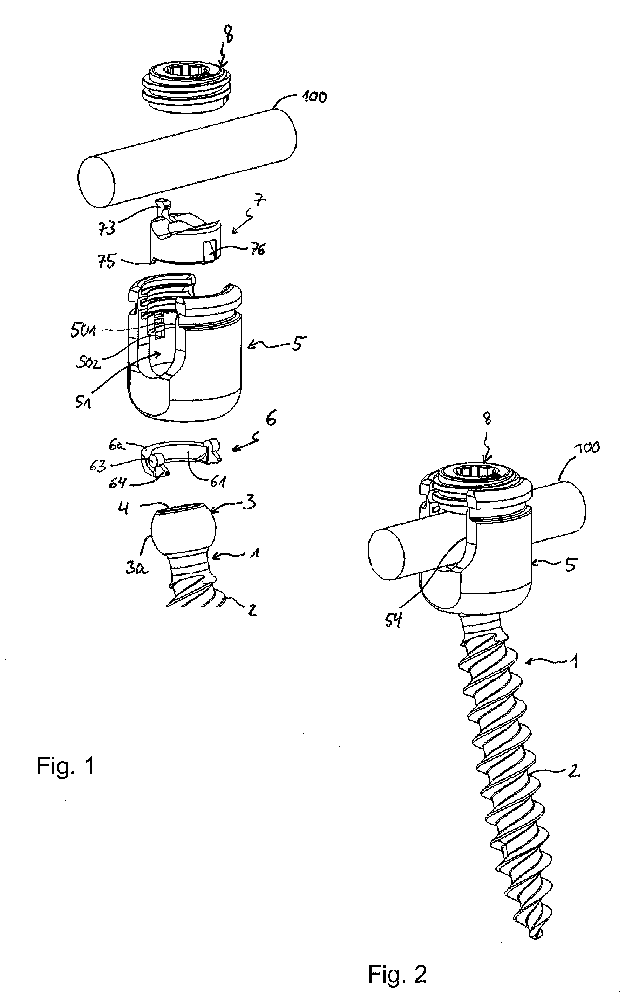

Referring to Figs. 1 to 3, a bone anchoring device includes a bone anchoring element 1 having a shank 2 for anchoring in bone or in a vertebra and a head 3. The shank 2 has a bone engagement structure, such as a bone thread. The head 3 may be shaped as a segment of a sphere, thus comprises a spherically-shaped outer surface portion 3a that may include a region with the greatest outer diameter of the head 3. In addition, the head 3 comprises at its free end and engagement portion 4 for engagement with a tool, such as a driver.

Further, the bone anchoring device includes a coupling device for coupling the bone anchoring element 1 to a rod 100 which is configured to connect at least two or more bone anchoring devices. The coupling device includes a receiving part 5, a retainer element 6 arranged in the receiving part 5 and a pressure element 7 for exerting pressure onto the head 3 of the bone anchoring element 1 and for providing support for the rod 100. To lock the head 3 in the rod 100 in the receiving part 5, a locking element 8 in the form of an inner screw or set-screw may be provided.

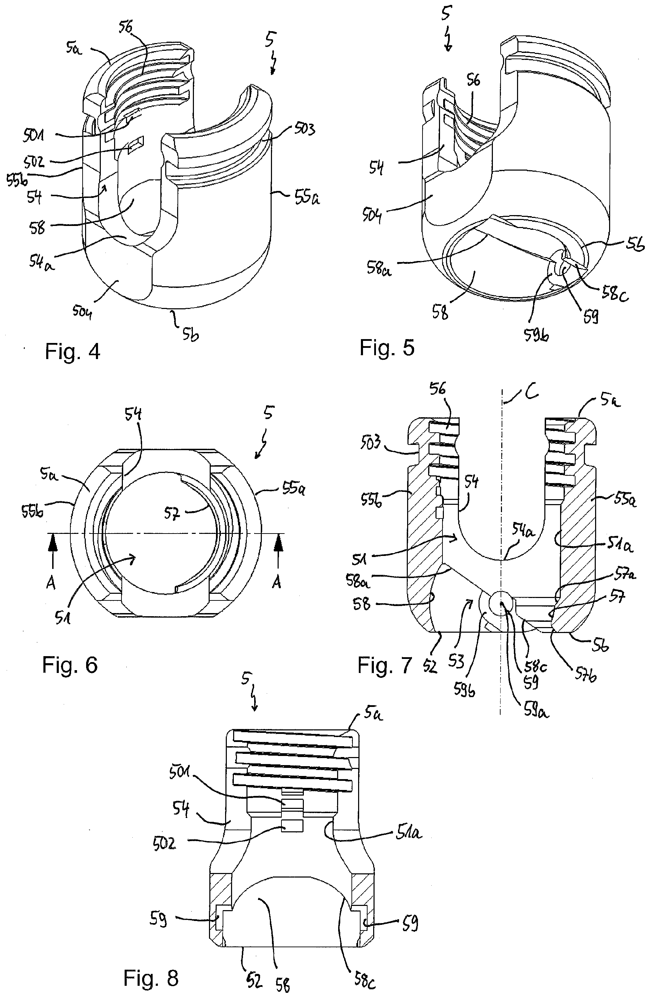

Referring in addition to Figs. 4 to 8, the receiving part 5 will be explained in greater detail. The receiving part 5 has a first or top end 5a and second or bottom end 5b opposite to the top end 5a. In general, the receiving part 5 may have a substantially cylindrical outer shape with a central longitudinal axis C extending through the top end 5a and the bottom end 5b. Coaxially to the central axis C, a passage 51 is provided that extends from the top end 5a to the bottom end 5b and forms a lower opening 52 at the bottom end 5b. At a distance from the top end 5a, the passage 51 widens into an accommodation space 53 in which the retainer element 6 is arranged. The accommodation space 53 is configured to receive the head 3 and at least a portion of the pressure element 7 therein. The receiving part 5 further comprises a substantially U-shaped recess 54 starting at the top end 5a and extending in a direction of the bottom end 5b and having a bottom 54a. By means of the substantially U-shaped recess 54, two free legs 55a, 55b are formed defining between them a channel that is open towards the top end 5a for receiving the rod 100. At an inner surface of the legs 55a, 55b, an internal thread 56 is formed, which is in the exemplary embodiment a square thread or another flat thread. The accommodation space 53 is asymmetric regarding the portion below the legs 55a, 55b in the direction towards the bottom end 5b, respectively.

As can be seen in particular in Fig. 7, a portion of the passage 51 below the internal thread 56 is formed by a substantially cylindrical bore 51a that is sized so as to receive a portion of the pressure element 7 slidably therein. In the region of the internal thread, the passage may have a slightly larger inner diameter than that of the coaxial bore 51a. On the side of the leg 55a, at a distance from the bottom end 5b, an inner wall portion of the receiving part 5 in the accommodation space 53 provides a first seat portion 57 for an inserted head 3. A circumferential extension of the first seat portion 57 may be slightly smaller than half of the circumference of the accommodation space 53. In greater detail, the first seat portion 57 may extend from a circumferential position close to the center of the channel formed by the U-shaped recess 54 on one side of the leg 55a up to a circumferential position close to the center of the channel on the other side of the leg 55a. The first seat portion 57 may have a spherical shape matching the spherical outer surface portion 3a of the head 3. However, other shapes may be possible which allow the head 3 to pivot in the first seat portion 57. In the axial direction, the bore 51a may narrow towards the first seat portion 57 with a narrowing transition portion 57a. Furthermore, a small bevelled surface 57b may be provided between the first seat portion 57 and the bottom end 5b. It should be mentioned that the first seat portion 57 is monolithic with the receiving part 5 or at least is fixedly connected to the receiving part.

The other side of the accommodation space 53, i.e. the side below the opposite leg 55b in the direction of the bottom end 5b, is configured to house the retainer element 6. In greater detail, the retainer element 6 is receivable in the accommodation space in a swivel-mounted manner. To achieve this, the receiving part comprises a spherical recess 58 that starts at the bottom end 5b and extends into the coaxial bore 51a up to a distance from the bottom 54a of the U-shaped recess 54. The border of the spherical recess 58 has the shape of a portion of a circle that extends in the receiving part 5 in an inclined manner and forms an edge 58a in the inner wall of the accommodation space 53 extending in an upwardly inclined manner in the direction towards the top end 5a. The edge 58a provides a first abutment for the swiveling movement of the retainer element 6 towards the top end 5a. A size of the spherical recess 58 corresponds to an outer surface of the retainer element 6 such that the retainer element 6 is configured to slide along the inner wall of the spherical recess 58 which will be explained further below.

Two opposite cylindrical recesses 59 with center points 59a are formed in the inner wall of the accommodation space 53 as best seen in Figs. 7 and 8. The cylindrical recesses 59 serve for receiving corresponding projections of the retainer element 6 to permit the retainer element 6 to swivel around an axis defined by the two opposite center points 59a. The center points 59a of the recesses 59 are located at a circumferential position corresponding to ends of the rod channel and at an axial position slightly above the first seat portion 57. Thus, the swivel axis for the retainer element is parallel to a longitudinal axis of the U-shaped recess 54. As can be seen in particular in Fig. 8, the recesses 59 extend from the coaxial bore 51a into the wall of the spherical recess 58. To allow the retainer element 6 to swivel around the center points 59a of the cylindrical recesses 59, shallow flat portions 59b extend around the cylindrical recesses 59 towards the bottom end 5b, respectively.

A border of the spherical recess 58 between the cylindrical recesses 59 and the bottom end 5b, respectively, provides an edge 58c that forms a continuation of the edge 58a up to the bottom end 5b. The edge 58c forms a second abutment for the retainer element 6 when the retainer element 6 is in a lowermost position.

In addition, a first recess or first pocket 501 may be provided in the inner wall of the leg 55b that is on the side of the spherical recess 58 for the retainer element 6. The first pocket 501 may be positioned at the center of the leg 55b in the circumferential direction and at an axial position below the internal thread 56. A circumferential length of the first pocket 501 may be such that a portion of the pressure element 7 can be received therein such that the pressure element is secured against rotation. In addition, the first pocket 501 may provide a stop or an abutment for restricting an upward movement of the pressure element 7 towards the top end 5a when the pressure element 7 is assembled with the receiving part 5 and when the pressure element 7 is in an insertion position. Between the first pocket 501 and the bottom 54a of the U-shaped recess 54 a second pocket 502 in a circumferential position below the first pocket 501 is formed for engagement with a portion of the pressure element 7 to secure a pre-locking position of the pressure element 7. A portion of the inner wall between the pockets may be rounded to enable the pressure element 7 to slide from the first pocket 501 into the second pocket 502.

Moreover, in the outer surface of each of the legs 55a, 55b at a distance from the top end 5a, a circumferential tool engagement groove 503 may be formed for engagement with a tool or an instrument. At an outer surface of the receiving part 5 aligned with the U-shaped recess 54, two opposite planar areas 504 may be provided that can reduce an overall width of the receiving part 5.

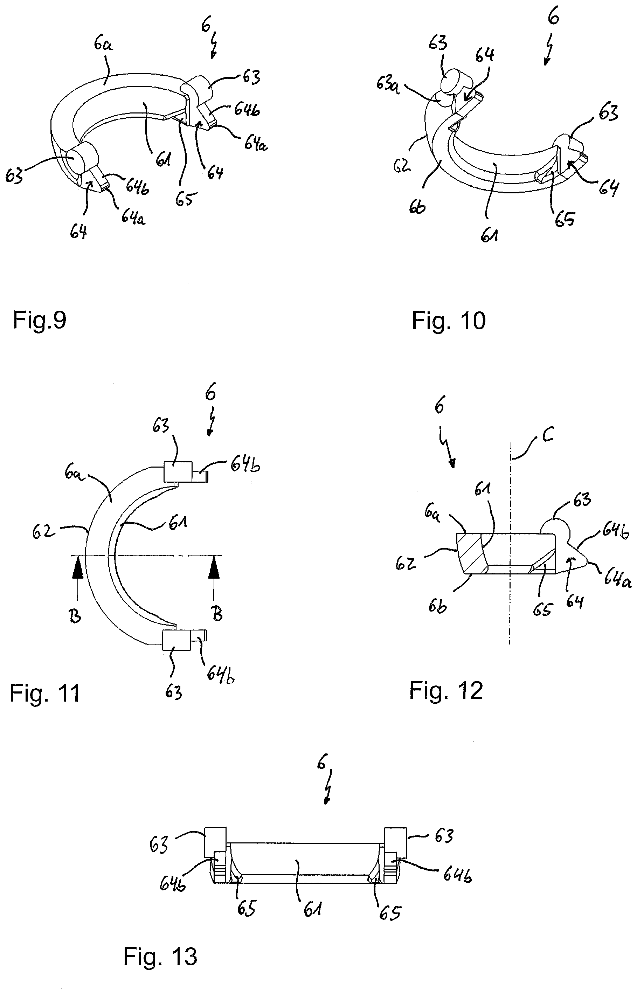

Referring to Figs. 9 to 13, the retainer element 6 will be described. The retainer element 6 has an overall appearance of a half ring. In greater detail, the retainer element 6 has an upper end 6a and an opposite lower end 6b both of which are substantially planar surfaces. An inner spherically-shaped surface provides in the mounted configuration a second seat portion 61 for the head 3 of the bone anchoring element 1. An outer spherically-shaped surface 62 is configured to slide along the inner surface of the spherical recess 58 of the accommodation space 53. At the ends of the seat portion 61 in the circumferential direction cylindrical pins 63 are formed that are configured to engage the cylindrical recesses 59 of the receiving part, respectively, to provide a swivel connection for the retainer element 6 in the receiving part 5. Thus, when the retainer element 6 is in the receiving part 5 and the pins 63 of the retainer element 6 engage the recesses 59 of the receiving part 5, the retainer element 6 is configured to swivel in the receiving part 5 around an axis defined by the center points 59a of the cylindrical recesses 59.

Two triangular projections 64 with substantially flat side walls project outward from the pins 63 in a direction perpendicular to the cylinder axis of the pins 63. The tips 64a of the triangular projections form the outermost points of the retainer element 6. An upper inclined surface 64b of the triangular projections is configured to abut against the edge 58c in the receiving part that is formed between the cylindrical recesses 59 and the bottom end 5b. As can be seen in particular in Fig. 10 the outer side of the triangular projections 64 are recessed from the spherical outer surface 62. Around the pins 63, a shallow countersink-like flat area 63a may be provided. This countersink-like area 63a together with the shallow recess 59b around the cylindrical recesses 59 in the receiving part 5 permits a swiveling motion of the retainer element 6 in the receiving part 5 without substantial friction. In the circumferential direction of the retainer element 6 at the inner side between the triangular projections 64 and the second seat portion 61, two opposite triangular recesses 65 are provided that allow the head 3 to enter the half ring of the retainer element 6 when the retainer element 6 is in the receiving part 5. It shall be noted, that preferably the retainer element 6 is a monolithic piece.

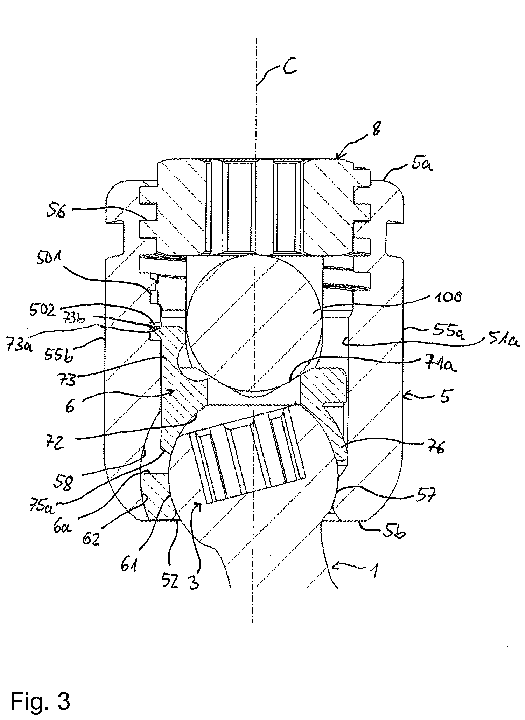

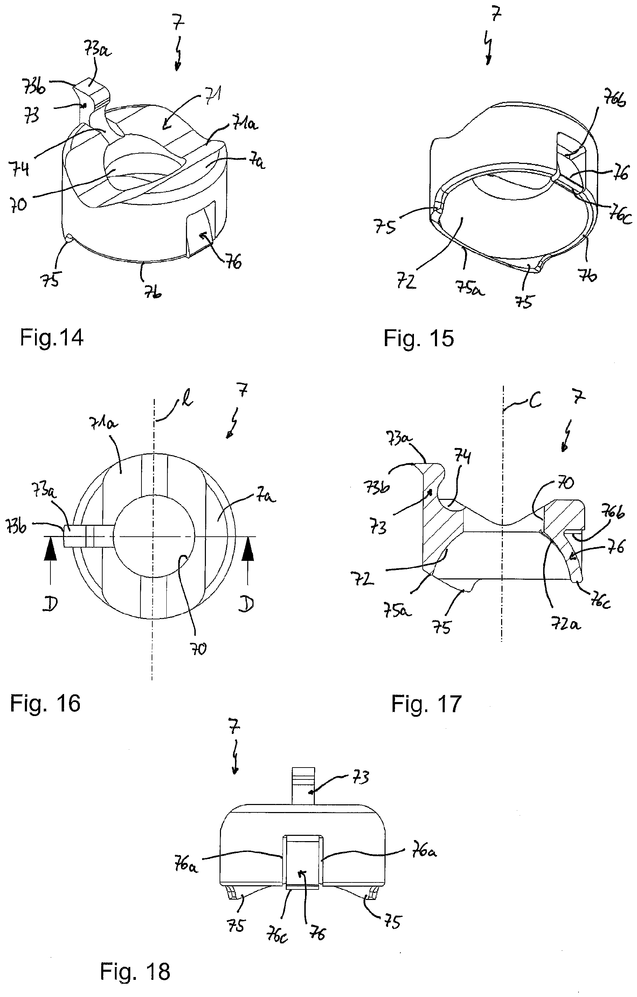

Referring further to Figs. 14 to 18, the pressure element 7 will be described. Preferably, the pressure element is a monolithic piece which is configured to be arranged in the passage 51 of the receiving part 5. The pressure element 7 is substantially cylindrical and comprises an upper end 7a and an opposite lower end 7b. Adjacent to the upper end 7a, a rod receiving recess 71 is formed that provides a rod support surface 71a. The rod support surface 71a may have a substantially V-shaped cross-section with a longitudinal axis l extending substantially perpendicular to a cylinder axis of the pressure element 7 that coincides with the central axis C of the receiving part 5 when the pressure element 7 is arranged in the receiving part 5. The depth of the rod receiving recess 71 may be smaller than a diameter of the rod 100. Hence, when the rod 100 rests on the rod support surface 71a, the rod 100 may project over the upper end 7a of the pressure element 7 as shown, for example, in Fig. 3. The V-shape of the rod support surface 71a allows the use of rods with different diameters.

A substantially spherical recess 72 is formed at the lower end 7b of the pressure element 7. The spherical recess 72 is configured to matingly receive an upper portion of the head 3 of the bone anchoring element 1 as shown, for example, in Fig. 3. At one side of the rod support surface 71a an arm 73 extends out of the upper end 7a of the pressure element 7. The arm 73 comprises a preferably flat upper surface 73a and an outwardly extending rim 73b. The outwardly extending ring 73b is configured to engage the first pocket 501 or the second pocket 502 at the inner wall of the receiving part 5. A recess 74 is formed in the upper end 7a between the arm 73 and the rod support surface 71a. By means of this, the arm 73 is slightly flexible such that the outer rim 73b can disengage from the first pocket 501 and engage the second pocket 502 of the receiving part 5.

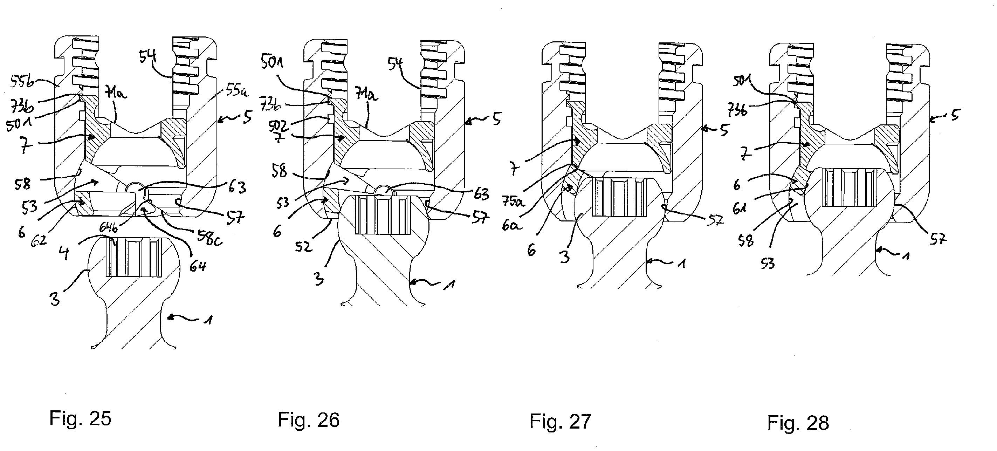

The lower end 7b of the pressure element comprises at one side of the longitudinal axis l of the rod support surface 71a and symmetrical with respect to the circumferential position of the arm 73 two spaced apart projecting portions 75. The shape of the projecting portions may be substantially triangular. An edge 75a formed between the tips of projecting portions 75 has a contour that fits to the upper end 6a of the retainer element 6 when the pressure element 7 and the retainer element 6 are in the receiving part and the retainer element 6 is at an uppermost position as for example shown in Fig. 28.

Moreover, the pressure element 7 comprises at the other side of the longitudinal axis of the rod support surface, i.e. opposite to the arm 73, a flexible wall portion or flexible finger 76 that is spaced apart from the rest of the cylindrical wall by two slits 76a. The flexible finger 76 may be convexly curved. It extends from an upper end of the spherical recess 72 into a recess 76b formed between the slits 76a and has a reduced thickness in the radial direction compared to the rest of the cylindrical wall. As can be seen in particular in Figs. 15, 17 and 18, a lower edge 76c of the flexible finger 76 may extend slightly below the lower end 7b of the pressure element 7. An inner spherical surface 72a of the flexible finger 76 has a reduced diameter compared to the spherical recess 72. When the head 3 is received in the spherical recess 72, the flexible finger 76 clamps the head 3 by friction since the inner surface 72a of the flexible finger 76 is slightly offset towards the inside of the spherical recess 72.

In addition, the pressure element 7 comprises a coaxial bore 70 for allowing access to the head 3 with a tool or an instrument such as a driver.

Parts and portions of the bone anchoring device may be made of any material, preferably, however, of titanium or stainless steel or a bio-compatible metal or metal alloy, or a plastic material. For bio-compatible alloys, a NiTi alloy, for example Nitinol, may be used. Other materials that can be used are, for example, magnesium alloys and alloys, or bio-compatible plastic materials, for example polyetheretherketone (PEEK) or poly-L-lactide acid (PLLA). The parts can be made of the same or of different materials from one another.

A preferred method of manufacturing the bone anchoring device includes manufacturing at least the receiving part 5 and/or the retainer element 6 and/or the pressure element 7 with an additive manufacturing method, more specifically, an additive layer manufacturing method. In such a method, the respective part is built-up by layer-wise deposition of a building material and solidifying or melting the building material in each layer at positions corresponding to the cross-section of the part in the respective layer. A suitable method is, for example, selective laser sintering (SLS) or selective laser melting (SLM) in which the building material is a powder, such as metal powder or a plastic powder, and a laser is used to melt the powder. Consecutive layers of the building material are applied to a support surface or a previous layer, and the laser beam is steered to the positions of a layer which correspond to the cross-section of the respective part in each layer. Thus, the receiving part is build-up in an additive manner. The building material may preferably be a titanium powder or a stainless steel powder or a suitable metal alloy powder. Alternatively, an electron beam may be used to melt the building material. Also, other methods of powder-based three-dimensional printing in which layers of a powder material are deposited and solidified by applying a binder material at positions corresponding to the respective part may be used. Still further additive manufacturing methods that may be employed include, for example, fused deposition modelling (FDM) or additive lithography-based metal manufacturing (LMM).

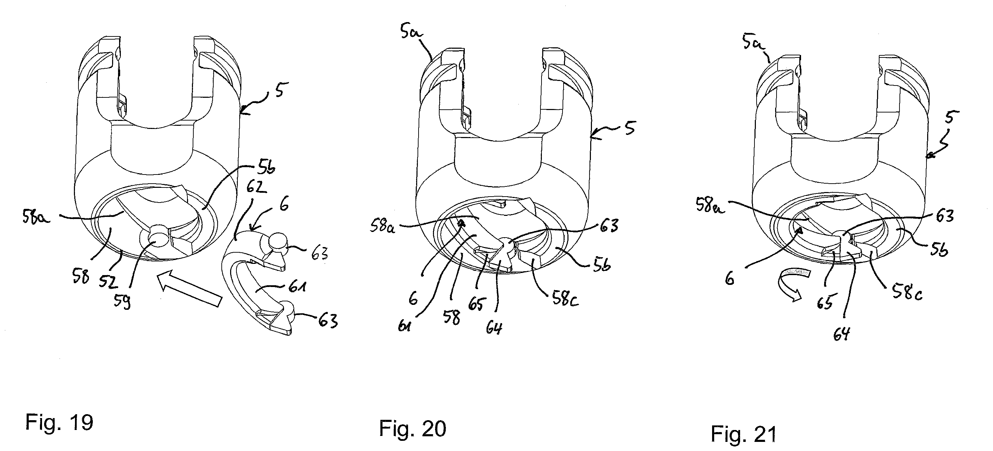

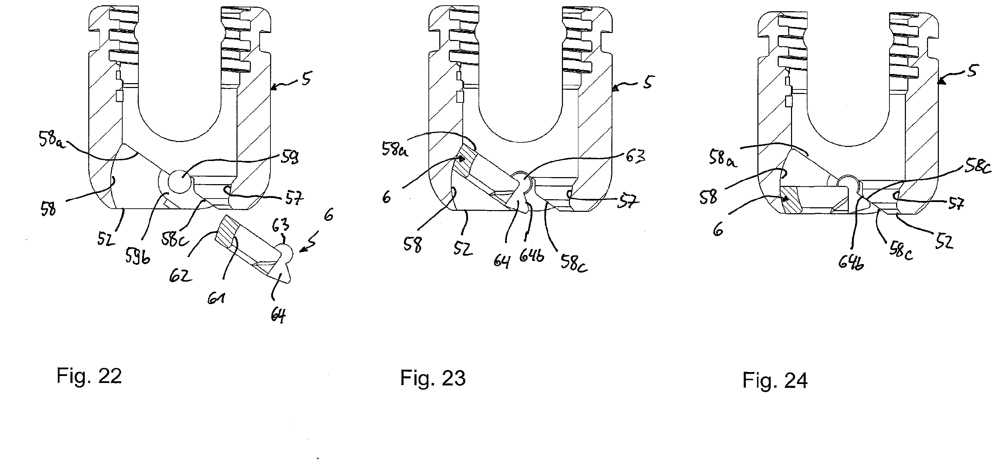

Referring to Figs. 19 to 24, mounting the retainer element 6 to the receiving part 5 will be explained. As shown in Fig. 19 and 22 the retainer element 6 is oriented with the outer spherical surface 62 facing the bottom end 5b of the receiving part 5. The retainer element 6, which is due to its half-ring shape slightly flexible, is slightly compressed and inserted into the receiving part 5 through the lower opening 52 until the pins 63 snap into the cylindrical recesses 59 of the receiving part 5.

When the pins 63 are in the recesses 59, the retainer element 6 can swivel within the receiving part 5 around the axis defined by the center points 59a of the recesses 59 or, in other words, around the cylinder axis of the pins 63. When the retainer ring 6 swivels, it slides with the outer spherical surface portion 62 along the inner spherical surface portion of the recess 58 in the accommodation space 53 of the receiving part 5. The swiveling motion of the retainer element 6 is confined by an uppermost and a lowermost position of the retainer element 6.

As depicted in Fig. 20 and 23, an uppermost position of the retainer element 6 is defined in that the upper end 6a of the retainer element 6 abuts against the edge 58a in the inner wall of the accommodation space 53.

As shown in Figs. 25 and 24, a lowermost position of the retainer element 6 is defined in that the upper surface 64b of the triangular projections 64 of the retainer element 6 abuts against the edge 58c in the receiving part 5.

Next, steps of mounting the coupling device to the bone anchoring element 1 are described referring to Figs. 25 to 28. The coupling device is pre-assembled with the receiving part 5 comprising the retainer element 6 and also the pressure element 7 which has the rod support surface 71a aligned with the U-shaped recess 54

As shown in Fig. 25, the outer rim 73b of the arm 73 of the pressure element 7 engages the first pocket 501 in the inner wall of the leg 55b of the receiving part 5. This is the insertion position in which the pressure element 7 is prevented from moving further towards the top end 5a of the receiving part 5. It shall be noted that the pocket 501 also provides a securing structure for securing the rotational position of the pressure element 7 in the receiving part 5. The retainer element 6 may be in the lowermost position in which the upper surface 64b of the triangular projections 64 abuts against the edge 58c in the receiving part. The outer spherical surface 62 contacts the inner spherical surface of the spherical recess 58 in the receiving part. The coupling device is oriented with the bottom end 5b towards the head 3.

As shown in Fig. 26, the head 3 is inserted through the lower opening 52 into the receiving part 5. Thereby, the retainer element 6 is moved upward by swiveling around the cylinder axis of the pins 63.

As shown in Fig. 27, when the head 3 is further inserted into the accommodation space 53, the retainer element 6 is moved further upwards until its upper end 6a abuts against the edge 58a in the receiving part 5. At the same time, the edge 75a at the lower end 7b of the pressure element 7 abuts against the upper surface 6a of the retainer element 6.

Next, as shown in Fig. 28, the head 3 enters between the first seat portion 57 provided at the inner wall of the accommodation space 53 and the second seat portion 61 of the retainer element 6. The recesses 65 at the inner side of the retainer element 6 permit the head 3 to enter the retainer element 6. In this position, the lower edge 75a between the projections 75 of the pressure element 7 lies on the upper surface 6a of the retainer element 6.

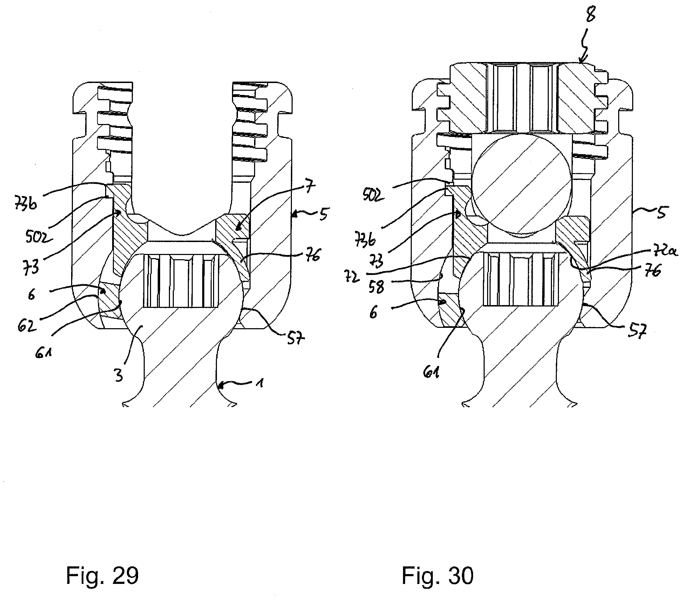

Referring to Fig. 29, the pressure element 7 is moved downward, such that the outer rim 73a of the arm 73 moves out of the first pocket 501 and snaps into the second pocket 502. At the same time, the projections 75 move the retainer element 6 downward. Once the retainer element 6 is at a position below the portion with a greatest outer diameter E of the head 3 the retainer element 6 narrows the lower opening 52 of the receiving part 5 such that the head 3 cannot be removed through the lower opening 52. Thus, the pressure element 7 is secured in a pre-locking position in which removal of the head 3 through the lower opening 52 is no longer possible.

The flexible finger 76 of the pressure element 7 clamps the head 3 by means of friction prior to final locking of the head 3. The flexible finger may also be designed such that it also contacts the inner wall of the receiving part 5 such that the clamping force that acts on the head may be increased when the flexible finger 76 is compressed inwards. The angular position of the bone anchoring element 1 relative to the receiving pat 5 can be changed by pivoting the receiving part 5 with respect to the bone anchoring element 1 to overcome the frictional force.

Finally, as shown in Fig. 30, once a particular angular position of the bone anchoring element 1 with respect to the receiving part 5 has been set, the rod 100 is inserted and the locking element 8 is screwed between the legs 55a, 55b and tightened to lock the angular position. Thereby, the pressure element 7 presses the head 3 into the seat provided by the first seat portion 61 of the retainer element 6 and the second seat portion 57 provided at the inner wall of the receiving part 5.

In the clinical use, the bone anchoring device can be used in a first manner in which the bone anchoring element 1 is pre-assembled with the coupling device comprising the receiving part 5, the retainer element 6 and the pressure element 7 outside a patient's body or in second manner in which the bone anchoring element 1 is already inserted in a bone or in a vertebra and the coupling device is mounted *in-situ* onto the head 3 of the bone anchoring element 1. Usually, a plurality of polyaxial bone anchoring devices are inserted into bone parts or into vertebrae, in particular into the pedicles of vertebrae. The receiving parts 5 are then aligned so that the rod 100 can be received in the U-shaped recess 54 of two or more of the bone anchoring devices.

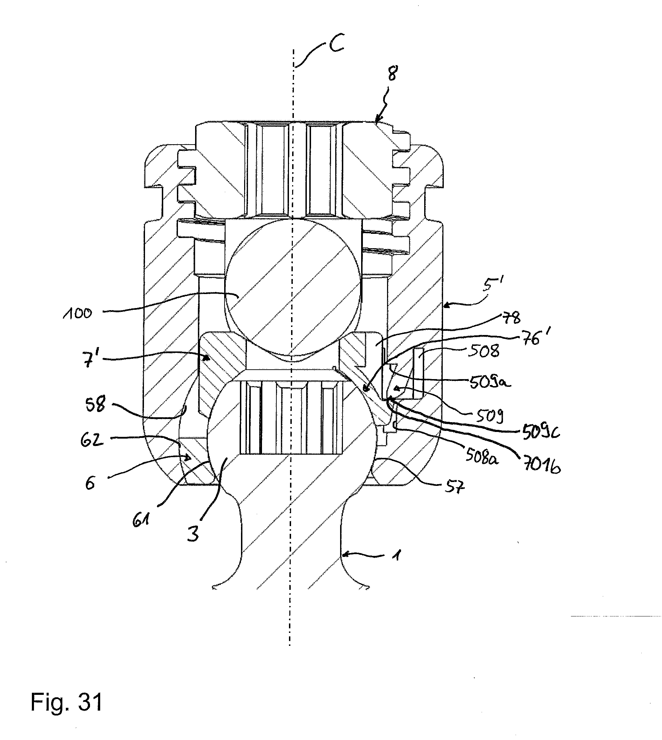

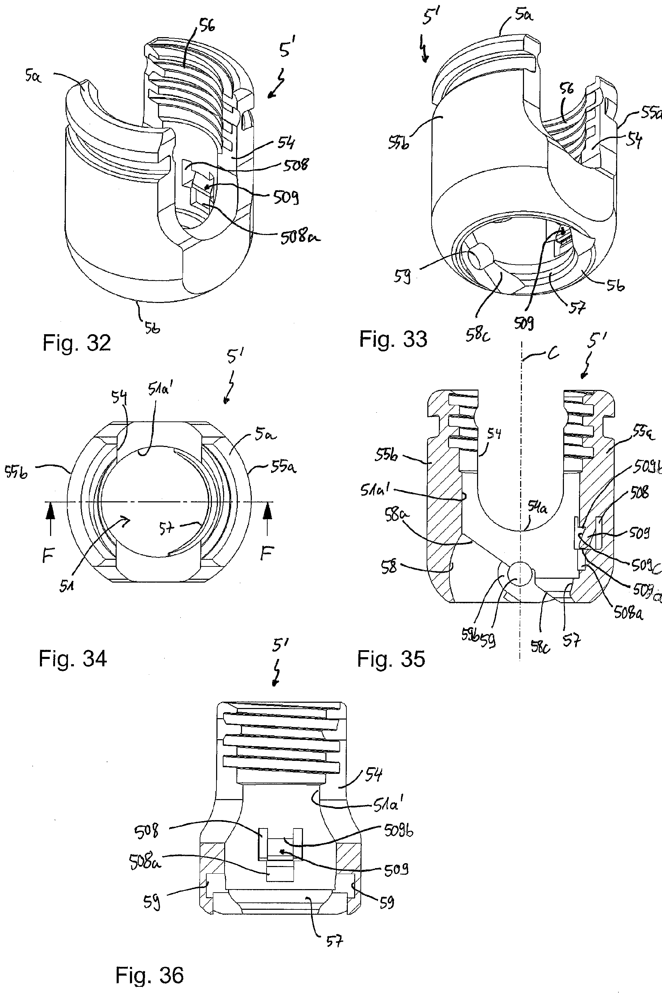

A second embodiment of the bone anchoring device will be described with reference to Figs. 31 to 45. Parts and portions of the second embodiment that are identical or highly similar to those of the first embodiment according to Figs. 1 to 30 are indicated with the same reference numerals and the description thereof will not be repeated.

The coupling device according to the second embodiment differs from the coupling device according to the first embodiment in the design of the receiving part and of the pressure element. The retainer element 6 and the structure for receiving the retainer element 6 in the receiving part are the same as in the first embodiment.

The receiving part 5' will be described referring in particular to Figs. 31 to 36. The coaxial bore 51a' of the passage 51 is slightly oval in such a manner that the longer inner diameter is oriented in the direction of the U-shaped recess 54. Correspondingly, the outer shape of the pressure element 7' is slightly oval so that it fits in the oval coaxial bore 51a'. By means of this, the pressure element 7' can be secured against rotation in the receiving part 5'. Due to the oval outer contour, the pressure element 7' can be mounted only in the orientation in which the rod support surface 71a and the U-shaped recess 54 are already aligned.

The receiving part 5' further lacks the first and the second pocket 501, 502 on the side of the leg 55b. To enable the pressure element 7' to assume an insertion position and a pre-locking position, the receiving part 5' comprises a substantially rectangular first recess 508 in the inner wall of the coaxial bore 51a'. The first recess 508 is located in a circumferential direction in the middle of the leg 55a and extends axially from a position slightly above the bottom 54a of the U-shaped recess 54 to a position above the cylindrical recesses 59. A second shallower and narrower recess 508a may be provided below the first rectangular recess 508 in the direction towards the bottom end 5b. The recesses 508, 508a are on the side of the receiving part 5' that is opposite to the spherical recess 58 for the retainer element 6. A flexible wall portion or flexible finger 509 is formed such that it can flex to a slight extent into the first rectangular recess 508. To achieve this, the flexible finger 509 is at its upper end connected to the wall of the receiving part 5' and extends in an inclined manner in the direction of the inside of the bore 51a'. A free end 509a of the flexible finger 509 is located in the rectangular first recess 508 immediately above the second recess 508a. On the upper end of the flexible finger 509 a step 509b is formed. The step 509b forms a first stop for defining an insertion position of the pressure element 7'. Furthermore, the free end 509a of the flexible finger 509 forms a second stop for defining a pre-locking position of the pressure element 7'. The inclined flexible finger 509 provides a ramp 509c along which a portion of the pressure element 7' can slide.

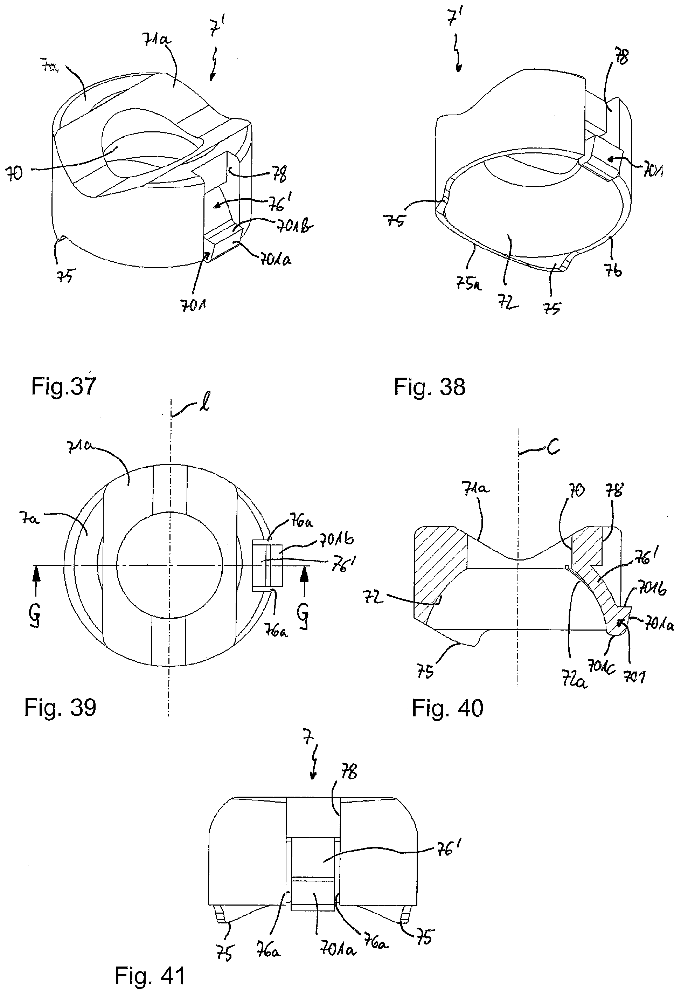

Referring to Figs. 37 and 41, the pressure element 7' will be explained. The pressure element 7' differs from the pressure element 7 in that it lacks the arm 73. Hence, the upper end 7a is flat on both sides of the rod support surface 71a.

The pressure element 7' also comprises a flexible wall portion or flexible finger 76' in its outer wall. The flexible finger 76' differs from the flexible finger 76 of the first embodiment by the shape of the free end. In greater detail, the flexible finger 76' comprises a thickened end portion 701 with an upwardly inclined outer side surface 701a and a substantially flat upper surface 701b. The inclined outer side surface 701a and the flat upper surface 701b of the end portion 700 protrude from the outer contour of the pressure element 7' as best seen in Figs. 37 and 40. A lower end 701c of the end portion 701 may be rounded. The lower end 701c extends slightly below the lower end 7b of the pressure element 7'. The upwardly inclined outer side surface 701a is configured to engage the ramp 509c formed by the inclined flexible finger 509 of the receiving part 5'. The flat upper surface 701b is configured to engage the step portion 509b of the receiving part 5' for securing an insertion position of the pressure element 7. Further, the flat upper surface 701b is configured to engage the free end 509a of the flexible finger 509 of the receiving part 5' for securing a pre-locking position of the pressure element 7'. As is the first embodiment, the flexible finger 76' is offset from the spherical recess towards the inside, such that its inner wall 72a is configured to clamp an inserted head by friction.

Since the upwardly inclined outer side surface 701a extends radially beyond the outer surface of the pressure element 7', the flexible finger 76' may be slightly compressed when the pressure element 7'is in the receiving part 5'. This may increase the friction force acting on the head 3.

In addition, the pressure element 7' comprises a recess 78 that extends from the upper end 7a up to the flexible finger 76'. The recess 78 permits to access the flexible finger 76' with an instrument to press on the outer surface of the flexible finger 76' to disengage the flexible finger 76' from either the step portion 509b or from the end portion 509a of the flexible finger 509 of the receiving part 5' to release the pressure element 7' from the insertion position or the pre-locking position.

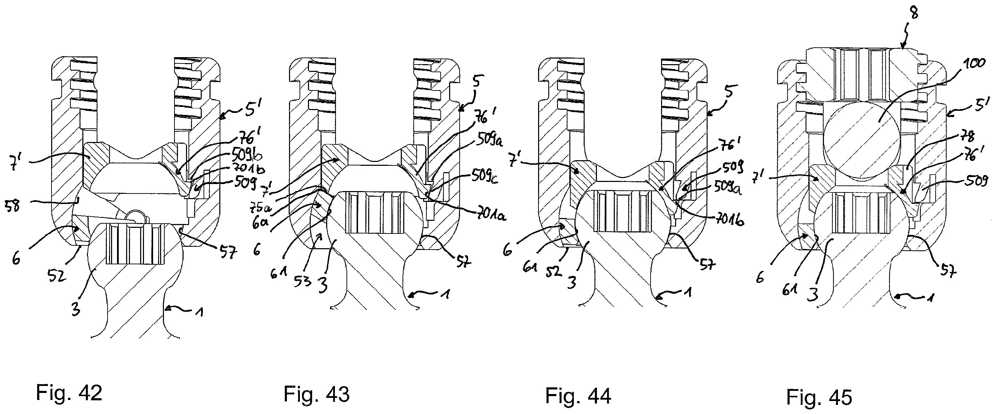

Referring to Figs. 42 to 45, steps of mounting the coupling device of the second embodiment to the head 3 of the bone anchoring element 1are described. As shown in Fig. 42, the pressure element 7' and the retainer element 6 are mounted to the receiving part 5'. The pressure element 7' is in an insertion position, in which the flat upper surface 701b of the end portion of the flexible finger 76' is below the step portion 509b and prevented from moving upwards by the step portion 509b. The retainer element 6 is in a lowermost position. The head 3 of the bone anchoring element enters into the accommodation space thereby moving the retainer element 6 around the swivel axis.

As shown in Fig. 43, when the head 3 has entered the accommodation space 53, it is held between the first seat portion 57 in the receiving part 5' and the second seat portion 61 in the retainer element 6. The retainer element 6 is in the uppermost position in which its upper surface 6a abut against the edge 58 in the receiving part. At the same time, the pressure element 7' contacts with its lower edge 75a the upper surface 6a of the retainer element 6. When the pressure element 7' is moved downward, the upwardly inclined outer side surface 701a of the flexible finger 76' slides along the ramp 509c formed by the flexible finger 509 of the receiving part 5'.

Next, as depicted in Fig. 44, when the pressure element 7' has been moved further downward until the flat upper surface 701b engages the free end 509a of the flexible finger 76', the pressure element 7' is in the pre-locking position. At the same time, the retainer element 6 has been moved downward by the projections 75 at the lower end 7b of the pressure element 7' so that the retainer element 6 narrows the lower opening 52 to prevent inadvertent removal of the head 3 through the lower opening 52.

**In** the configuration shown in Fig. 44, the head 3 is clamped by friction via the flexible finger 76 and simultaneously the pressure element 7' is in a pre-locking position. Thus, the cooperation of the flexible finger 509 of the receiving part 5' and the flexible finger 76' of the pressure element 7' provides for both, a frictional clamping of the head and pre-locking.

Finally, as shown in Fig. 45, the rod is inserted and the locking element 8 is tightened. Thereby, the pressure element 7' further presses onto the head 3 which in turn presses the head 3 into the seat formed by the first seat portion 57 of the receiving part 5' and the second seat portion 61 of the retainer element 6 to lock the angular position of the bone anchoring element 1 relative to the receiving part 5'.

Modification of the bone anchoring device may be conceivable. The shape of the parts is not limited to the detailed shape as shown in the figures. Deviations may be possible and encompassed by the disclosure. The first embodiment may also have an oval bore 51a and a pressure element with oval outer contour. Instead of one arm 73 two opposite arms, one on either side of the rod support surface 71a may be provided in the pressure element 7 of the first embodiment and the receiving part may comprise pockets 501, 502 on both sides. Instead of two projections 75 at the lower end 7b of the pressure element 7, 7', a single projection may be sufficient.

Instead of the locking member 8 being a set screw all other kinds of locking assemblies known in the art may be used. For the bone anchoring element all types of bone anchoring elements that are suitable for anchoring in bone or a vertebra, such as bone screws, bone nails, etc. may be used.

As used in the present specification and the appended claims, the term "rod" shall be understood as including any elongate member, regardless of the cross-sectional shape of the elongate member. Specifically, a spinal stabilization rod as used herein may have a substantially circular, oval, or angular cross-section. Such a cross-section may further vary along a length of the rod. The rod may be stiff or flexible.

Moreover, the seat for the head may have a design that allows to pivot the bone anchoring element to a greater pivot angle to one side compared to other sides. The head and the seat may be shaped so as to allow pivoting only in one single plane or in several discrete planes.

## Claims

1. A coupling device for coupling a rod to bone or to a vertebra, the coupling device including
a receiving part (5, 5') configured to be pivotably connectable to a head (3) of a bone anchoring element (1), the receiving part (5, 5') having a first end (5a) and a second end (5b), a central axis (C) extending through the first end (5a) and the second end (5b), a channel (54) for receiving a rod (100) and an accommodation space (53) for accommodating the head (3), the accommodation space (53) having a lower opening (52) at the second end (5b), an inner surface portion of the receiving part (5, 5') in the accommodation space providing a first seat portion (57) for the head (3); and
a retainer element (6) configured to be arranged in the accommodation space (53) and having an inner surface providing a second seat portion (61) for the head (3);
wherein the first seat portion (57) of the receiving part (5) and the second seat portion (61) of the retainer element (6) form a seat that permits the head (3) to pivot relative to the receiving part (5, 5').

2. The coupling device of claim 1, wherein the retainer element (6) has a half-ring shape.

3. The coupling device of claim 1 or 2, wherein the first seat portion (57) and the second seat portion (61) are spherically-shaped.

4. The coupling device of on of claims 1 to 3, wherein the retainer element (6) is insertable in the accommodation space (53) through the lower opening (52) of the receiving part.

5. The coupling device of one of claims 1 to 4, wherein the retainer element (6) is supported in the receiving part (5, 5') such that it can swivel, preferably around an axis perpendicular to the central axis (C).

6. The coupling device of claim 5, wherein the retainer element (6) is configured to swivel between a first position which is defined by a first abutment (58a) and a second position which is defined by a second abutment (58c).

7. The coupling device of claim 6, wherein the retainer element (6) comprises two free end portions (64) which are shaped to abut against the second abutment (58c), preferably wherein the second abutment (58c) is an edge formed in an inner wall of the accommodation space (53) and the free end portions (64) comprise an upper surface (64b) abutting against the second abutment (58c) when the retainer element (6) is in the second position.

8. The coupling device of one of claims 1 to 7, wherein the retainer element (6) comprises at least one cutout (65) in its inner wall to allow a head (3) to enter the seat portion (61).

9. The coupling device of one of claims 1 to 8, wherein the retainer element (6) comprises a spherical outer surface portion (62) and wherein an inner wall portion (58) of the accommodation space (53) comprises a spherical portion mating the spherical outer surface (62) portion of the retainer element (6).

10. The coupling device of one of claims 1 to 9, further comprising a pressure element (7, 7') configured to exert pressure on the head (3), the pressure element (7, 7') being positionable partially in the accommodation space (53) and having a lower end (7b) which faces the retainer element (6) when the retainer element (6) and the pressure element (7, 7') are in the receiving part.

11. The coupling device of claim 10, wherein the pressure element (7, 7') comprises at least one projection (75) projecting from the lower end (7b) which projection (75) is configured to act on the retainer element (6).

12. The coupling device of claim 10 or 11, wherein the pressure element (7, 7') comprises a recess (72) for receiving a portion of the head (3) and a, preferably flexible, wall portion (76, 76') that is offset towards the inside of the recess (72) and that is configured to act on the head (3) to clamp the head (3) by means of friction.

13. The coupling device of claim 12, wherein an outer surface (701a) of the wall portion (76') provides an inclined ramp and wherein an inner wall of the accommodation space (53) comprises a wall portion (509) with an outer surface (509c) providing an inclined ramp along which the outer surface (701a) of the wall portion (76') of the pressure element (7') can slide, preferably wherein the wall portion (76') of the pressure element (7') and the wall portion (509) of the receiving part (5') can interact in such a manner that the pressure element (7') is secured against a movement towards the first end (5a), more preferably wherein the flexible wall portion (76') of the pressure element (7') can interact with the wall portion (509) of the receiving part (5') to define an insertion position and to define a pre-locking position.

14. The coupling device of claim 13, wherein the pressure element (7') comprises a recess for permitting an instrument to enter and compress the wall portion (76') towards the head (3) such that the pressure element can move towards the first end (5a) of the receiving part (5).

15. The coupling device of one of claims 10 to 14, wherein the pressure element (7, 7') is partially positioned in a passage (51, 51a) of the receiving part (5, 5') that extends from the first end (53) into the accommodation space (53), preferably the passage (51a') being non-cylindrical, more preferably oval-shaped, and the pressure element (7') having a corresponding non-cylindrical outer contour.

16. A bone anchoring device comprising the coupling device of one of claims 1 to 15 and a bone anchoring element (1) having a shank (2) for anchoring in bone and a head (3), preferably a head (3) with a spherical outer surface portion (3a).
